(19) Europäisches Patentamt
European Patent Office
Office européen des brevets

(11) Numéro de publication : **0 409 692 B1**

# (12) FASCICULE DE BREVET EUROPEEN

(45) Date de publication du fascicule du brevet : **09.03.94 Bulletin 94/10**

(51) Int. Cl.$^5$ : **C07D 277/82,** A61K 31/425, C07D 417/06, C07D 417/12

(21) Numéro de dépôt : **90401976.7**

(22) Date de dépôt : **09.07.90**

## (54) Dérivés d'imino-2 hétérocyclylalkyl-3 benzothiazoline, leur préparation et les médicaments les contenant.

(30) Priorité : **13.07.89 FR 8909483**
**11.05.90 FR 9005889**

(43) Date de publication de la demande : **23.01.91 Bulletin 91/04**

(45) Mention de la délivrance du brevet : **09.03.94 Bulletin 94/10**

(84) Etats contractants désignés : **AT BE CH DE DK ES FR GB GR IT LI LU NL SE**

(56) Documents cités :
**EP-A- 0 050 551**
**EP-A- 0 374 040**
**EP-A- 0 375 510**
**FR-A- 2 357 553**

(73) Titulaire : **RHONE-POULENC SANTE**
**20, avenue Raymond Aron**
**F-92160 Antony (FR)**

(72) Inventeur : **Jimonet, Patrick**
**13 avenue de Normandie**
**F-78 450 Villepreux (FR)**
Inventeur : **Le Blevec, Joseph**
**24 rue de la Fraternite**
**F-92 700 Colombes (FR)**
Inventeur : **Nemecek, Conception**
**25 Rue Rollin Regnier**
**F-94 600 Choisy Le Roi (FR)**

(74) Mandataire : **Pilard, Jacques et al**
**RHONE-POULENC SANTE, Service Brevets,**
**20 Avenue Raymond Aron**
**F-92165 Antony Cédex (FR)**

EP 0 409 692 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne des composés de formule :

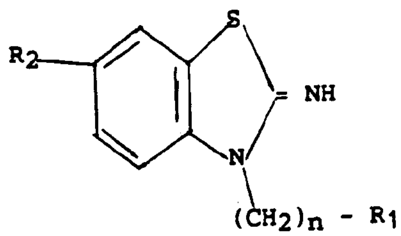

(I)

leurs sels, leurs procédés de préparation et les médicaments les contenant.

Dans la formule (I),

- $R_1$ représente
  - . un radical pipérazinyl-1 substitué en position -4 par un radical (a) phényle, (b) un radical phényle substitué par au moins un substituant choisi parmi les atomes d'halogène, les radicaux alkyle et alcoxy, (c) un radical phénylalkyle, (d) un radical pyridyle ou (e) un radical pyrimidinyle,
  - . un radical tétrahydro-1,2,3,6 pyridyl-1 substitué en position -4 par un radical phényle ou phényle substitué par au moins un substituant choisi parmi les atomes d'halogène, les radicaux alkyl et alcoxy,
  - . un radical pipéridino substitué en position -4 par un radical phényle ou phényle substitué par au moins un substituant choisi parmi les atomes d'halogène, les radicaux alkyle et alcoxy,
- $R_2$ représente un radical polyfluoroalcoxy,
- n est égal à 2 ou 3,

Dans les définitions qui précèdent et celles qui seront citées ci-après, les radicaux alkyle et alcoxy et les portions alkyle et alcoxy contiennent 1 à 4 atomes de carbone en chaîne droite ou ramifiée.

Les radicaux polyfluoroalcoxy sont de préférence les radicaux trifluorométhoxy, pentafluoroéthoxy, trifluoro-2,2,2 éthoxy ou tétrafluoro-1,1,2,2 éthoxy.

Les atomes d'halogène sont de préférence les atomes de fluor, de chlore et de brome.

L'invention concerne également les sels d'addition des composés de formule (I) avec les acides minéraux ou organiques.

Les composés de formule (I) peuvent être préparés par hydrolyse d'un dérivé de formule :

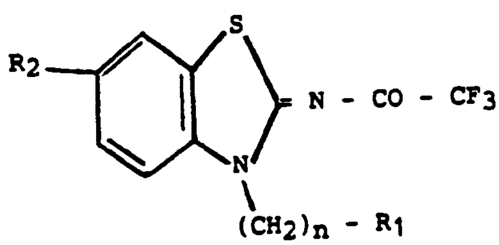

(II)

dans laquelle $R_1$, $R_2$ et n ont les mêmes significations que dans la formule (I).

Cette hydrolyse s'effectue généralement au moyen d'une base telle qu'un carbonate de métal alcalin (sodium, potassium de préférence) ou l'ammoniaque concentrée, au sein d'un mélange eau-alcool, à une température voisine de 20°C.

Les dérivés de formule (II) peuvent être obtenus par action d'un dérivé de formule :

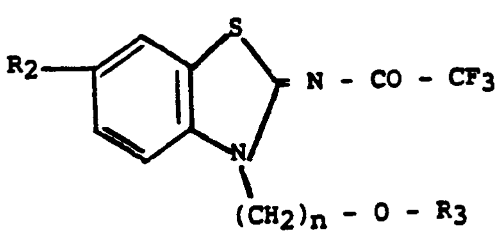

(III)

dans laquelle $R_2$ et n ont les mêmes significations que dans la formule (I) et $R_3$ représente un groupe réactif tel qu'un radical méthanesulfonyle ou p-toluènesulfonyle, sur une amine de formule :

$HR_1$ (IV)

dans laquelle $R_1$ a les mêmes significations que dans la formule (I).

Cette réaction s'effectue généralement au sein d'un solvant inerte tel qu'un solvant aromatique (benzène, toluène, xylène par exemple) ou le diméthylformamide, à une température comprise entre 20°C et la température d'ébullition du solvant.

Les dérivés de formule (III) peuvent être préparés par action d'un dérivé de formule :

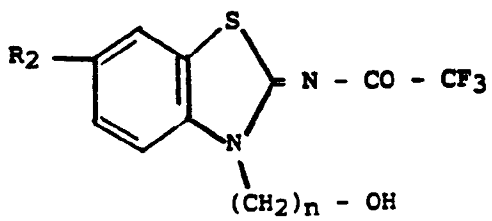

(V)

dans laquelle $R_2$ et n ont les mêmes significations que dans la formule (I), sur le chlorure d'acide méthanesulfonique ou p-toluènesulfonique, soit au sein d'un solvant inerte tel qu'un solvant aromatique (benzène, toluène, xylène par exemple) ou un solvant chloré (chloroforme, chlorure de méthylène par exemple), en présence d'une amine tertiaire telle que la triéthylamine, à une température voisine de 20°C, soit au sein de la pyridine, à une température voisine de 0°C.

Les dérivés de formule (V) peuvent être obtenus par action de trifluoroacétate d'éthyle sur un dérivé de formule :

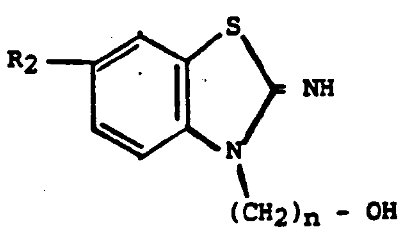

(VI)

dans laquelle $R_2$ et n ont les mêmes significations que dans la formule (I).

Cette réaction s'effectue généralement au sein d'un alcool (méthanol, éthanol par exemple), en présence d'une base tertiaire telle que la triéthylamine, à une température voisine de 20°C.

Les dérivés de formule (VI) peuvent être préparés par action d'un dérivé halogéné de formule :

$Hal - (CH_2)_n - OH$ (VII)

dans laquelle n a les mêmes significations que dans la formule (I) et Hal représente un atome d'halogène (brome ou chlore de préférence) sur un amino-2 polyfluoroalcoxy-6 benzothiazole.

Cette réaction s'effectue au sein d'un alcool (éthanol, méthanol de préférence), à la température d'ébullition du solvant.

Les amino-2 polyfluoroalcoxy-6 benzothiazoles peuvent être préparés par application ou adaptation de la méthode décrite par L.M. YAGUPOL'SKII et coll., Zh. Obshch. Khim., 33(7), 2301, (1963).

Les composés de formule (I) peuvent également être préparés par action de brome et d'un thiocyanate de métal alcalin sur un dérivé de formule :

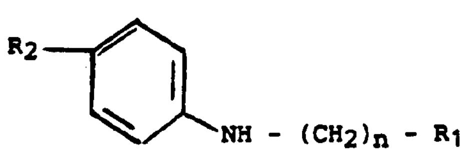

(VIII)

dans laquelle $R_1$, $R_2$ et n ont les mêmes significations que dans la formule (I).

Cette réaction s'effectue de préférence au sein de l'acide acétique, à une température voisine de 20°C.

Comme thiocyanate de métal alcalin, on utilise de préférence le thiocyanate de potassium.

Les dérivés de formule (VIII) peuvent être obtenus par action d'une amine de formule (IV) sur un dérivé de formule :

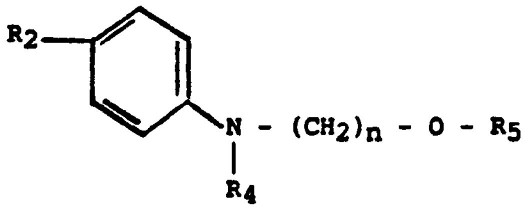

(IX)

dans laquelle $R_2$ et n ont les mêmes significations que dans la formule (I) et $R_4$ et $R_5$ représentent un radical p-toluènesulfonyle.

Cette réaction s'effectue de préférence en présence d'hydrogénocarbonate de sodium, au sein d'un solvant inerte tel que le diméthylformamide, à une température comprise entre 50°C et 100°C.

Les dérivés de formule (IX) peuvent être obtenus par action de chlorure de p-toluènesulfonyle sur un dérivé de formule :

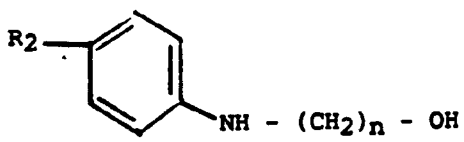

(X)

dans laquelle $R_2$ et n ont les mêmes significations que dans la formule (I).

Cette réaction s'effectue généralement au sein d'un solvant inerte tel qu'un solvant chloré (chloroforme, chlorure de méthylène par exemple), à une température comprise entre 0°C et 30°C.

Les dérivés de formule (X) peuvent être obtenus par action d'une polyfluoroalcoxy-4 aniline sur un dérivé de formule (VII).

Cette réaction s'effectue généralement à une température comprise entre 100°C et 170°C.

Les mélanges réactionnels obtenus par les divers procédés décrits précédemment sont traités suivant des méthodes classiques physiques (évaporation, extraction, distillation, cristallisation, chromatographie...) ou chimiques (formation de sels...).

Les composés de formule (I), sous forme de base libre, peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) et leurs sels présentent des propriétés pharmacologiques intéressantes. Ces composés sont actifs vis-à-vis des convulsions induites par le glutamate et sont donc utiles dans le traitement et la prévention des phénomènes convulsifs, des troubles schizophréniques et notamment des formes déficitaires de la schizophrénie, des troubles du sommeil, des phénomènes liés à l'ischémie cérébrale ainsi que des affections neurologiques où le glutamate peut être impliqué telles que la maladie d'Alzheimer, la maladie d'Huntington, la sclérose amyoptrophique latérale et l'atrophie olivopontocérébelleuse.

L'activité des composés de formule (I) vis-à-vis des convulsions induites par le glutamate a été déterminée selon une technique inspirée de celle de I.P. LAPIN, J. Neural. Transmission, vol. 54, 229-238, (1982) ; l'injection du glutamate par voie intracérébroventriculaire étant effectuée selon une technique inspirée de celle de R. CHERMAT et P. SIMON, J. Pharmacol. (Paris), vol. 6, 489-492 (1975). Leur $DE_{50}$ est généralement égale ou inférieure à 10 mg/kg.

Les composés de formule (I) présentent une toxicité faible. Leur $DL_{50}$ est supérieure à 15 mg/kg par voie I.P. chez la souris.

Pour l'emploi médicinal, il peut être fait usage des composés de formule (I) tels quels ou à l'état de sels pharmaceutiquement acceptables, c'est-à-dire non toxiques aux doses d'utilisation.

Comme exemples de sels pharmaceutiquement acceptables, peuvent être cités les sels d'addition avec les acides minéraux ou organiques tels que acétate, propionate, succinate, benzoate, fumarate, maléate, oxalate, méthanesulfonate, iséthionate, théophyllinacétate, salicylate, phénolphtalinate, méthylène-bis-β-oxynaphtoate, chlorhydrate, sulfate, nitrate et phosphate.

Les exemples suivants, donnés à titre non limitatif, montrent comment l'invention peut être mise en pratique.

EXEMPLE 1

1,3 g de {[(pyridyl-2)-4 pipérazinyl-1]-2 éthyl}-3 trifluoroacétylimino-2 trifluorométhoxy-6 benzothiazoline en solution dans un mélange de 19 $cm^3$ d'une solution aqueuse de carbonate de potassium à 7 % et 32 $cm^3$ de méthanol sont agités à une température voisine de 20°C pendant 4 heures. Le milieu est concentré sous pression réduite (20 mm de mercure ; 2,7 kPa) et le résidu est repris par 300 $cm^3$ d'eau distillée. La phase aqueuse est extraite par 3 fois 200 $cm^3$ d'éther éthylique, séchée sur sulfate de magnésium et concentrée à sec sous pression réduite. Le résidu obtenu est purifié par chromatographie sur colonne de silice avec de l'acétone comme éluant. Après formation du trichlorhydrate par addition d'éther chlorhydrique 4,2N dans l'acétone, on obtient 0,73 g de trichlorhydrate d'imino-2 {[(pyridyl-2)-4 pipérazinyl-1]-2 éthyl}-3 trifluorométhoxy-6 benzothiazoline fondant vers 230°C.

La {[(pyridyl-2)-4 pipérazidyl-1]-2 éthyl}-3 trifluoroacétylimino-2 trifluorométhoxy-6 benzothiazoline peut être préparée selon le procédé suivant : un mélange de 4,52 g de méthane sulfonate de (trifluoroacétylimino-2 trifluorométhoxy-6 benzothiazolinyl-3)-2 éthyl dans 100 $cm^3$ de toluène et de 6,9 g de (pyridyl-2) pipérazine est chauffé 2 heures à reflux, refroidi à 4°C puis essoré. Le filtrat toluènique est évaporé sous pression réduite et purifié par chromatographie sur colonne de silice avec du dichlorométhane comme éluant. On obtient 1,3 g de {[(pyridyl-2)-4 pipérazinyl-1]-2 éthyl}-3 trifluoroacétylimino-2 trifluorométhoxy-6 benzothiazoline fondant à 132°C.

Le méthane sulfonate de (trifluoroacétylimino-2 trifluorométhoxy-6 benzothiazolinyl-3)-2 éthyle peut être préparé de la façon suivante : 6,9 g de triéthylamine sont ajoutés progressivement à un mélange de 120 $cm^3$ de chlorure de méthylène, de 7,34 g de chlorure de l'acide méthane sulfonique et de 12 g de (trifluoroacétylimino-2 trifluorométhoxy-6 benzothiazolinyl-3)-2 éthanol. Après 1 heure d'agitation à une température voisine de 20°C, le milieu réactionnel est refroidi à 10°C, essoré, lavé avec 20 $cm^3$ de chlorure de méthylène froid, puis séché à 40°C sous pression réduite. On obtient 10 g de méthanesulfonate de (trifluoroacétylimino-2 trifluorométhoxy-6 benzothiazolinyl-3)-2 éthyle fondant à 145°C. Un 2ème jet de 2,7 g est obtenu par lavage du filtrat avec 100 $cm^3$ d'eau, séchage sur sulfate de magnésium, concentration jusqu'à un volume résiduel de 50 $cm^3$ environ, refroidissement à 5°C puis essorage.

Le (trifluoroacétylimino-2 trifluorométhoxy-6 benzothiazolinyl-3)-2 éthanol peut être préparé de la manière suivante : 20,7 g de bromhydrate d'(imino-2 trifluorométhoxy-6 benzothiazolinyl-3)-2 éthanol, 9,8 g de trifluoroacétate d'éthyle et 16,1 $cm^3$ de triéthylamine sont agités dans 100 $cm^3$ d'éthanol pendant 22 heures à une température voisine de 20°C. Après concentration à sec sous pression réduite, le résidu obtenu est purifié par chromatographie sur colonne de silice avec de l'acétate d'éthyle comme éluant. On obtient 19,2 g de (trifluoroacétylimino-2 trifluorométhoxy-6 benzothiazolinyl-3)-2 éthanol fondant à 144°C.

L'(imino-2 trifluorométhoxy-6 benzothiazolinyl-3)-2 éthanol peut être préparé selon le procédé suivant : 9,4 g d'amino-2 trifluorométhoxy-6 benzothiazole et 10 g de bromo-2 éthanol dans 30 $cm^3$ d'éthanol absolu sont chauffés pendant 95 heures à ébullition. Le mélange est ensuite refroidi à une température voisine de 20°C. Le précipité formé est filtré et lavé avec 100 $cm^3$ d'éther éthylique. On obtient 6,4 g de bromhydrate d'(imino-2 trifluorométhoxy-6 benzothiazolinyl-3)-2 éthanol fondant à 219°C.

L'amino-2 trifluorométhoxy-6 benzothiazole peut être préparé selon la méthode décrite par L.M. YAGUPOL'SKII et Coll., Zh. Obshch. Khim, 33(7), 2301 (1963).

EXEMPLE 2

En opérant comme à l'exemple 1 mais à partir de 0,9 g de {[(méthyl-4 phényl)-4 pipérazinyl-1]-2 éthyl}-3 trifluoroacétylimino-2 trifluorométhoxy-6 benzothiazoline dans 18 $cm^3$ de méthanol et de 14 $cm^3$ d'une solution hydroalcoolique à 7 % de carbonate de potassium, on obtient 0,7 g de l'imino-2 {[(méthyl-4 phényl)-4 pipérazinyl-1]-2 éthyl}-3 trifluorométhoxy-6 benzothiazoline fondant à 120°C.

La {[(méthyl-4 phényl)-4 pipérazinyl-1]-2 éthyl}-3 trifluoroacétylimino-2 trifluorométhoxy-6 benzothiazoline peut être préparée de la façon suivante : on opère comme à l'exemple 1, à partir de 4,52 g de méthane sulfonate de (trifluoroacétylimino-2 trifluorométhoxy-6 benzothiazolinyl-3)-2 éthyle dans 150 $cm^3$ de toluène et de 5,29 g de (méthyl-4 phényl)-4 pipérazine. Après purification par chromatographie sur colonne de silice avec un mélange de dichlorométhane et d'acétate d'éthyle (98-2 en volumes) comme éluant, on obtient 0,9 g de {[(méthyl-4 phényl)-4 pipérazinyl-1]-2 éthyl}-3 trifluoroacétylimino-2 trifluorométhoxy-6 benzothiazoline.

EXEMPLE 3

En opérant comme à l'exemple 1, à partir de 5,88 g de méthane sulfonate de (trifluoroacétylimino-2 trifluorométhoxy-6 benzothiazolinyl-3)-2 éthyle dans 200 $cm^3$ de toluène et de 6,87 g de (méthyl-2 phényl)-4

pipérazine et après 2 heures au reflux, le filtrat toluènique est évaporé sous pression réduite. Au résidu ainsi obtenu on ajoute 100 $cm^3$ de méthanol et 20 $cm^3$ d'une solution hydroalcoolique de carbonate de potassium à 7 % et agite 2 heures à environ 20°C. L'alcool est évaporé sous pression réduite et le résidu est purifié par chromatographies sur colonne de silice avec un mélange dichlorométhane-acétate d'éthyle (70-30 en volumes) comme éluant puis un mélange acétate d'éthyle-cyclohexane (50-50 en volumes) comme éluant. On obtient ainsi 1,8 g d'imino-2 {[(méthyl-2 phényl)-4 pipérazinyl-1]-2 éthyl}-3 trifluorométhoxy-6 benzothiazoline fondant à 135°C.

EXEMPLE 4

En opérant comme à l'exemple 1, mais à partir de 3,8 g de [(benzyl-4 pipérazinyl-1)-2 éthyl]-3 trifluoroacétylimino-2 trifluorométhoxy-6 benzothiazoline dans 20 $cm^3$ de méthanol et de 10 $cm^3$ d'une solution hydroalcoolique à 7 % de carbonate de potassium et après purification par chromatographie sur colonne de silice avec un mélange acétate d'éthyle-cyclohexane (50-50 en volumes) comme éluant puis addition d'éther chlorhydrique 4,2N, on obtient 2,5 g du chlorhydrate de [(benzyl-4 pipérazinyl-1)-2 éthyl]-3 imino-2 trifluorométhoxy-6 benzothiazoline fondant vers 230°C.

La [(benzyl-4 pipérazinyl-1)-2 éthyl]-3 trifluoroacétylimino-2 trifluorométhoxy-6 benzothiazoline peut être préparée de la façon suivante : on opère comme à l'exemple 1, à partir de 4,52 g de méthane sulfonate de (trifluoroacétylimino-2 trifluorométhoxy-6 benzothiazolinyl-3)-2 éthyle dans 120 $cm^3$ de toluène et de 7 g de benzyl-4 pipérazine. Après purification par chromatographie sur colonne de silice avec un mélange dichlorométhane-acétate d'éthyle (50-50 en volumes) comme éluant, on obtient 3,8 g de [(benzyl-4 pipérazinyl-1)-2 éthyl]-3 trifluoroacétylimino-2 trifluorométhoxy-6 benzothiazoline.

EXEMPLE 5

En opérant comme à l'exemple 1, à partir de 4,52 g de méthane sulfonate de (trifluoroacétylimino-2 trifluorométhoxy-6 benzothiazolinyl-3)-2 éthyle dans 150 $cm^3$ de toluène et de 6,5 g de (pyrimidinyl-2)-4 pipérazine et après 4 heures au reflux, le filtrat toluènique est évaporé sous pression réduite. 100 $cm^3$ de méthanol et 20 $cm^3$ d'une solution hydroalcoolique de carbonate de potassium à 7 % sont ajoutés au résidu toluènique. Après 2 heures d'agitation à environ 20°C le méthanol est évaporé à pression réduite et le résidu est purifié par chromatographie sur colonne de silice avec un mélange dichlorométhane-méthanol (90-10 en volumes) comme éluant. Après formation du trichlorhydrate par addition d'éther chlorhydrique 4,2N, on obtient 1,13 g de trichlorhydrate d'imino-2 {[(pyrimidinyl-2)-4 pipérazinyl-1]-2 éthyl}-3 trifluorométhoxy-6 benzothiazoline fondant vers 270°C.

EXEMPLE 6

3,6 g de chlorhydrate, de [(phényl-4 pipérazinyl-1)-3 propyl]-3 trifluoroacétylimino-2 trifluorométhoxy-6 benzothiazoline en solution dans un mélange de 45 $cm^3$ d'une solution aqueuse de carbonate de potassium à 7 % et 150 $cm^3$ de méthanol sont agités pendant 4 heures à une température voisine de 20°C. Après concentration à sec, le milieu réactionnel est repris dans 100 $cm^3$ d'eau distillée et la phase organique extraite par 2 fois 100 $cm^3$ d'acétate d'éthyle, séchée sur sulfate de magnésium et concentrée à sec sous pression réduite (20 mm de mercure ; 2,7 kPa). Après formation du dichlorhydrate par addition de 4 $cm^3$ d'éther chlorhydrique 4,2 N dans l'acétate d'éthyle, le précipité est recristallisé dans un mélange éthanol absolu-éther éthylique (50-50 en volumes). On obtient 1,6 g de dichlorhydrate d'imino-2 [(phényl-4 pipérazinyl-1)-3 propyl]-3 trifluorométhoxy-6 benzothiazoline fondant à 260°C.

Le chlorhydrate de [(phényl-4 pipérazinyl-1)-3 propyl]-3 trifluoroacétylimino-2 trifluorométhoxy-6 benzothiazoline peut être préparé selon le procédé suivant : 4,4 g de p-toluènesulfonate de (trifluoroacétylimino-2 trifluorométhoxy-6 benzothiazolinyl-3)-3 propyle, 1,44 g de N-phényl pipérazine et 0,68 g d'hydrogénocarbonate de sodium dans 50 $cm^3$ de diméthylformamide sont chauffés à 80°C pendant 19 heures. Le milieu réactionnel est concentré à sec sous pression réduite (7 mm de mercure ; 0,95 kPa), le résidu repris dans 100 $cm^3$ d'eau distillée et la phase organique extraite par 50 $cm^3$ de dichlorméthane. Après séchage sur sulfate de magnésium et concentration à sec sous pression réduite, le résidu huileux obtenu est repris par 15 $cm^3$ d'acide chlorhydrique aqueux 1N et le chlorhydrate formé précipité dans 15 $cm^3$ d'eau distillée. On obtient 4 g de chlorhydrate de [(phényl-4 pipérazinyl-1)-3 propyl]-3 trifluoroacétylimino-2 trifluorométhoxy-6 benzothiazoline fondant à 238°C.

Le p-toluènesulfonate de (trifluoroacétylimino-2 trifluorométhoxy-6 benzothiazolinyl-3)-3 propyle peut être préparé selon le procédé suivant : 11 g de (trifluoroacétylimino-2 trifluorométhoxy-6 benzothiazolinyl-3)-3 propanol sont ajoutés progressivement à 10,8 g de chlorure de p-toluènesulfonyle en solution dans 200 $cm^3$ de pyridine refroidie à 0°C. La réaction est poursuivie 1 heure à 5°C, puis le milieu réactionnel est gardé au froid (6-7°C) pendant 15 heures. Après addition à 2 litres d'eau distillée, extraction par 200 $cm^3$ d'acétate d'éthyle, lavage par 2 fois 50 $cm^3$ d'acide chlorhydrique 1N, séchage sur sulfate de magnésium et concentration à sec sous pression réduite, on obtient 6,7 g de p-toluènesulfonate de (trifluoroacétylimino-2 trifluorométhoxy-6 benzothiazolinyl-3)-3 propyle fondant à 139°C.

Le (trifluoroacétylimino-2 trifluorométhoxy-6 benzothiazolinyl-3)-3 propanol peut être préparé de la façon suivante : 24,8 g d'(imino-2 trifluorométhoxy-6 benzothiazolinyl-3)-3 propanol, 14,2 g de trifluoroacétate d'éthyle et 8,6 g de triéthylamine sont agités dans 250 $cm^3$ d'éthanol absolu pendant 24 heures à une température voisine de 20°C. Le milieu réactionnel est alors refroidi à 0-5°C et le précipité formé filtré et séché. On obtient 28,2 g de (trifluoroacétylimino-2 trifluorométhoxy-6 benzothiazolinyl-3)-3 propanol fondant à 144°C.

L'(imino-2 trifluorométhoxy-6 benzothiazolinyl-3)-3 propanol peut être préparé selon le procédé suivant : 82 g d'amino-2 trifluorométhoxy-6 benzothiazole et 65 $cm^3$ de bromo-3 propanol dans 25 $cm^3$ d'éthanol absolu sont chauffés pendant 72 heures à ébullition. Le mélange est ensuite refroidi à une température voisine de 20°C. L'huile obtenue est reprise dans 1 litre d'eau distillée et la phase organique extraite par 3 fois 200 $cm^3$ de dichlorométhane. Après séchage sur sulfate de magnésium et concentration à sec sous pression réduite, le produit brut est purifié par chromatographie sur colonne de silice avec de l'acétate d'éthyle comme éluant. On obtient 25 g d'(imino-2 trifluorométhoxy-6 benzothiazolinyl-3)-3 propanol fondant à 106°C.

## EXEMPLE 7

En opérant comme à l'exemple 1, à partir de 1 g de {[(m-tolyl)-4 pipérazinyl-1]-2 éthyl}-3 trifluoroacétylimino-2 trifluorométhoxy-6 benzothiazoline dans 30 $cm^3$ de méthanol et de 15 $cm^3$ d'une solution hydroalcoolique à 7 % de carbonate de potassium et après purification par chromatographie sur colonne de silice avec un mélange acétate d'éthyle-cyclohexane (50-50 en volumes) comme éluant puis addition d'éther chlorhydrique 4,2N, on obtient 0,6 g du chlorhydrate d'imino-2 {[(m-tolyl)-4 pipérazinyl-1]-2 éthyl}-3 trifluorométhoxy-6 benzothiazoline fondant vers 250°C.

La {[(m-tolyl)-4 pipérazinyl-1]-2 éthyl}-3 trifluoroacétylimino-2 trifluorométhoxy-6 benzothiazoline peut être préparée de la façon suivant : on opère comme à l'exemple 1, à partir de 4,52 g de méthane sulfonate de (trifluoroacétylimino-2 trifluorométhoxy-6 benzothiazolinyl-3)-2 éthyle dans 150 $cm^3$ de toluène et de 5,29 g de (méthyl-3 phényl)-4 pipérazine. Après purification par chromatographie sur colonne de silice avec un mélange dichlorméthane-acétate d'éthyle (70-30 en volumes) comme éluant, on obtient 1 g de {[(m-tolyl)-4 pipérazinyl-1]-2 éthyl}-3 trifluoroacétylimino-2 trifluorométhoxy-6 benzothiazoline.

## EXEMPLE 8

A un mélange de 2,62 g de phényl-4 [(trifluorométhoxy-4 anilino)-2 éthyl]-1 tétrahydro-1,2,3,6 pyridine et 2,8 g de thiocyanate de potassium dans 50 $cm^3$ d'acide acétique on ajoute goutte à goutte 1,15 g de brome en solution dans 15 $cm^3$ d'acide acétique à une température voisine de 20°C. Après traitement habituel, on obtient 0,67 g de dichlorhydrate d'imino-2 [(phényl-4 tétrahydro-1,2,3,6 pyridyl-1)-2 éthyl]-3 trifluorométhoxy-6 benzothiazoline fondant à 256°C.

La phényl-4 [(trifluorométhoxy-4 anilino)-2 éthyl]-1 tétrahydro-1,2,3,6 pyridine peut être préparée de la manière suivante : un mélange de 5,56 g de p-toluènesulfonate de N-p-toluènesulfonyl (trifluorométhoxy-4 anilino)-2 éthyle, 1,84 g de phényl-4 tétrahydro-1,2,3,6 pyridine et 0,97 g d'hydrogénocarbonate de sodium dans 95 $cm^3$ de diméthylformamide est chauffé 18 heures à 80°C. Après traitement habituel, on obtient 2,62 g de phényl-4 [(trifluorométhoxy-4 anilino)-2 éthyl]-1 tétrahydro-1,2,3,6 pyridine sous forme d'une huile utilisée telle quelle dans les synthèses ultérieures.

Le p-toluènesulfonate de N-p-toluènesulfonyl (trifluorométhoxy-4 anilino)-2 éthyle peut être préparé selon le procédé suivant : à 5,0 g de (trifluorométhoxy-4 anilino)-2 éthanol et 6,35 $cm^3$ de triéthylamine dans 50 $cm^3$ de dichlorméthane à 0°C, on ajoute progressivement 8,6 g de chlorure de p-toluènesulfonyle. La réaction est poursuivie 2 heures à une température voisine de 20°C, puis le milieu réactionnel lavé 3 fois par 50 $cm^3$ d'eau distillée et la phase organique séchée sur sulfate de magnésium et concentrée à sec sous pression réduite (20 mm de mercure ; 2,7 kPa). Après addition de 50 $cm^3$ d'éthanol absolu, le précipité formé est filtré. On obtient 7,3 g de p-toluènesulfonate de N-p-toluènesulfonyl (trifluorométhoxy-4 anilino)-2 éthyle fondant à 88°C.

Le (trifluorométhoxy-4 anilino)-2 éthanol peut être préparé de la manière suivante : 88,5 g de trifluorométhoxy-4 aniline et 31,2 g de bromo-2 éthanol sont chauffés à 160°C pendant 1,5 heures. Après refroidissement à une température voisine de 20°C, le milieu réactionnel est repris dans 200 $cm^3$ de dichlorométhane, l'insoluble filtré et le filtrat concentré à sec sous pression réduite. Après purification par chromatographie sur colonne

de silice avec un mélange acétate d'éthyle-cyclohexane (40-60 en volumes) comme éluant, on obtient 26,8 g de (trifluorométhoxy-4 anilino)-2 éthanol sous forme d'une huile orangée.

EXEMPLE 9

1,1g de chlorhydrate de [(phényl-4 pipérazinyl)-2 éthyl]-3 trifluoroacétylimino-2 trifluorométhoxy-6 benzothiazoline en solution dans un mélange de 5 cm3 d'une solution acqueuse de carbonate de potassium à 7% et 100 cm3 de méthanol sont agités à une température voisine de 20°C pendant 5 heures. Le milieu réactionnel est ajouté à 200 cm3 d'eau distillée et la phase organique extraite par 3 fois 150 cm3 d'éther éthylique, séchée sur sulfate de magnésium et concentrée à sec sous pression réduite (20 mm de mercure ; 2,7kPa). Après formation du chlorhydrate par addition de 0,45 cm3 d'éther chlorhydrique 4,2N dans 30 cm3 d'acétate d'éthyle, on obtient 0,8g de chlorhydrate d'imino-2 [(phényl-4 pipérazinyl)-2 éthyl]-3 trifluorométhoxy-6 benzothiazoline fondant à 230°C.

Le chlorhydrate de [(phényl-4 pipérazinyl)-2 éthyl]-3 trifluoroacétylimino-2 trifluorométhoxy-6 benzothiazoline peut-être préparé selon le procédé suivant : 6,5g de p-toluènesulfonate de (trifluoroacétylimino-2 trifluorométhoxy-6 benzothiazolinyl-3)-2 éthyle, 2,2g de N-phényl pipérazine et 1,0g de bicarbonate de sodium dans 80 cm3 de diméthylformamide sont chauffés à 60°C pendant 19 heures. Le milieu réactionnel est ajouté à 300 cm3 d'eau distillée et la phase organique extraite par 3 fois 50 cm3 de dichlorométhane. Après séchage sur sulfate de magnésium et concentration à sec sous pression réduite, le résidu huileux obtenu est repris par 20 cm3 d'acide chlorhydrique 1N et le chlorhydrate formé précipité dans 20 cm3 d'éthanol. On obtient 1,1g de chlorhydrate de [(phényl-4 pipérazinyl)-2 éthyl]-3 trifluoroacétylimino-2 trifluorométhoxy-6 benzothiazoline fondant à 204°C.

Le p-toluènesulfonate de (trifluoroacétylimino-2 trifluorométhoxy-6 benzothiazolinyl-3)-2 éthyle peut être préparé selon le procédé suivant : 19,3 g de (trifluoroacétylimino-2 trifluorométhoxy-6 benzothiazolinyl-3)-2 éthanol est ajouté progressivement à 19,7g de chlorure de p-toluènesulfonyle en solution dans 120 cm3 de pyridine refroidie à 0°C. La réaction est poursuivie 1 heure à 10-15°C. Le milieu réactionnel est ajouté à 500 cm3 d'eau distillée et la phase organique extraite par 3 fois 100 cm3 de dichlorométhane. Après lavage par 2 fois 50 cm3 d'acide chlorhydrique 1N, puis 2 fois 50 cm3 d'eau distillée, séchage sur sulfate de magnésium et concentration à sec sous pression réduite (20 mm de mercure ; 2,7kPa), on obtient 14,1g de p-toluènsulfonate de (trifluoroacétylimino-2 trifluorométhoxy-6 benzothiazolinyl-3)-2 éthanol fondant à 143°C.

EXEMPLE 10

1,53g de chlorhydrate de {[(méthoxy-4 phényl)-4 pipérazinyl-1]-2 éthyl}-3 trifluoroacétylimino-2 trifluorométhoxy-6 benzothiazoline, 33 cm3 de méthanol et 21 cm3 d'une solution à 7% de carbonate de potassium dans un mélange de 2 volumes de méthanol et de 5 volumes d'eau, sont agités 4 heures à uen température voisine de 20°C. Le milieu réactionnel est concentré sous pression réduite puis extrait à l'éther sulfurique. La phase organique est séchée sur sulfate de magnésium puis additionnée d'un excès de solution alcoolique d'acide chlorhydrique. Le précipité est essoré, lavé à l'éther sulfurique, séché à 50°C sous 20 mm de mercure. On obtient 1,05g de trichlorhydrate de l'imino-2 {[(méthoxy-4 phényl)-4 pipérazinyl-1]-2 éthyl}-3 trifluorométhoxy-6 benzothiazoline fondant à 240°C.

Le chlorhydrate de {[(méthoxy-4 phényl)-4 pipérazinyl-1]-2 éthyl}-3 trifluoroacétylimino-2 trifluorométhoxy-6 benzothiazoline peut être préparé de la manière suivante : Un mélange de 4,52 g de méthane sulfonate de (trifluoroacétylimino-2 trifluorométhoxy-6 benzothiazolinyl-3)-2 éthyle, de 120 cm3 de toluène et de la base libérée à partir de 10,56g de p-méthoxy phényl-4 pipérazine dichlorhydrate est chauffé 1 heure à reflux, refroidi à +4°C puis essoré. Le filtrat toluénique est évaporé sous pression réduite puis additionné de 45 cm3 de solution aqueuse d'acide chlorhydrique 1,2N. Le chlorhydrate se sépare sous forme amorphe puis cristallise par trituration avec 50 cm3 d'éthanol. Après essorage, lavage avec 15 cm3 d'éthanol et séchage à 50°C sous 20 mm de mercure, on obtient 1,7g de chlorhydrate de {[(méthoxy-4 phényl)-4 pipérazinyl-1]-2 éthyl}-3 trifluoroacétylimino-2 trifluorométhoxy-6 benzothiazoline.

EXEMPLE 11

En opérant comme à l'exemple 9 mais à partir de 1,4g de chlorhydrate de {[(diméthoxy-3,4-phényl)-4 pipérazinyl-1]-2 éthyl}-3 trifluoroacétylimino-2 trifluorométhoxy-6 benzothiazoline dans 30 cm3 de méthanol et de 19 cm3 d'une solution hydroalcoolique à 7% de carbonate de potassium, on obtient 0,97g de trichlorhydrate de l'imino-2 {[(diméthoxy-3,4 phényl)-4 pipérazinyl-1]-2 éthyl}-3 trifluorométhoxy-6 benzothiazoline fondant à 250°C.

Le chlorhydrate de {[(diméthoxy-3,4 phényl)-4 pipérazinyl-1]-2 éthyl]-3 trifluoroacétylimino-2 trifluorométhoxy-6 benzothiazoline, peut être préparé de la façon suivante : on opère comme à l'exemple 9, à partir de 4,3g de méthane sulfonate de (trifluoroacétylimino-2 trifluorométhoxy-6 benzothiazolinyl-3)-2 éthyle, de 70 cm3 de toluène et de la base libérée à partir de 14g de (diméthoxy-3,4-phényl)-4 pipérazine dichlorhydrate. On obtient 1,45g du chlorhydrate attendu fondant à 260°C.

EXEMPLE 12

En opérant comme à l'exemple 9 mais à partis de 1,7g de chlorhydrate de {[(fluoro-4 phényl)-4 pipérazinyl-1]-2 éthyl}-3 trifluoroacétylimino-2 trifluorométhoxy-6 benzothiazoline, de 36 cm3 de méthanol et de 21 cm3 de solution hydrométhanolique à 7% de carbonate de potassium. On obtient le dichlorhydrate de {[(fluoro-4 phényl)-4 pipérazinyl-1]-2 éthyl}-3 imino-2 trifluorométhoxy-6 benzothiazoline fondant à 260°C.

Le chlorhydrate de {[(fluoro-4 phényl)-4 pipérazinyl-1]-2 éthyl}-3 trifluoroacétylimino-2 trifluorométhoxy-6 benzothiazoline peut être préparé de la manière suivante : on opère comme à l'exemple 9, à partir de 9g de (fluoro-4 phényl)-4 pipérazine, de 70 cm3 de toluène et de 4,52g de méthane sulfonate de (trifluoroacétylimino-2 trifluorométhoxy-6 benzothiazolinyl-3)-2 éthyle. On obtient 1,7g de produit attendu fondant à 240°C.

EXEMPLE 13

En opérant comme à l'exemple 9 mais à partir de 1,40g de {[(chloro-4 phényl)-4 pipérazinyl-1]-2 éthyl}-3 trifluoroacétylimino-2 trifluorométhoxy-6 benzothiazoline, de 30 cm3 de méthanol et de 19 cm3 de solution hydrométhanolique à 7% de carbonate de potassium, on obtient 0,9g de dichlorhydrate de {[(chloro-4 phényl)-4 pipérazinyl-1]-2 éthyl}-3 imino-2 trifluorométhoxy-6 benzothiazoline fondant à 270°C.

La {[(chloro-4 phényl)-4 pipérazinyl-1]-2 éthyl}-3 trifluoroacétylimino-2 trifluorométhoxy-6 benzothiazoline peut être préparée de la manière suivante : on chauffe 1 heure et 15 minutes à reflux un mélange de 4,52g de méthane sulfonate de (trifluoroacétylimino-2 trifluorométhoxy-6 benzothiazolinyl-3)-2 éthyle, de 70 cm3 de toluène et de la base libérée à partir de 13,45g de (chloro-4 phényl)-4 pipérazine dichlorhydrate. Après refroidissement à 10°C et essorage du précipité, le filtrat toluénique est concentré puis purifié par chromatographie sur colonne de silica avec un mélange de cyclohexane et d'acétate d'éthyle (50-50 en volumes) comme éluant. Après recristallisation dans 50 cm3 d'éthanol, on obtient 1,4g du produit attendu fondant à 165°C.

EXEMPLE 14

3,0g de phényl-4 [(trifluorométhoxy-4 anilino)-2 éthyl]-1 pipéridine et 3,2g de thiocyanate de potassium dans 25 cm3 d'acide acétique sont traités goutte à goutte par 1,3 g de brome en solution dans 15 cm3 d'acide acétique à uen température voisine de 20°C. La réaction est poursuivie 18 heures à cette température. Après addition de 100 cm3 d'eau distillée, le milieu réactionnel est neutralisé par de la soude à 30% et la phase organique extraite par 3 fois 50 cm3 d'acétate d'éthyle, séchée sur sulfate de magnésium et concentrée à sec sous pression réduite (20 mm de mercure; 2,7kPa). La résidu obtenu est purifié par chromatographie sur colonne de silice avec de l'acétate d'éthyle comme éluant. Après formation du dichlorhydrate par addition de 4 cm3 d'éther chlorhydrique 4,2N dans 30 cm3 d'acétate d'éthyle, on obtient 2,4g de dichlorhydrate d'imino-2 [(phényl-4 pipéridino)-2 éthyl]-3 trifluorométhoxy-6 benzothiazoline sublimant vers 220°C.

La phényl-4 [(trifluorométhoxy-4 anilino)-2 éthyl]-1 pipéridine peut être préparée de la façon suivante: un mélange de 7,2g de p-toluènesulfonate de N-p-toluènesulfonyl (trifluorométhoxy-4 anilino)-2 éthyle, 4,9g de phényl-4 pipéridine et 2,4g d'hydrogénocarbonate de sodium dans 50 cm3 de diméthylformamide est chauffé 18 heures à 80°C. Après refroidissement à une température voisine de 20°C, le milieu réactionnel est concentré à sec sous pression réduite (7 mm de mercure; 0,95kPa). Le résidu est lavé 2 fois par 30 cm3 d'eau puis repris par 50 cm3 d'éthanol et concentré à sec sous pression réduite. Le produit brut est traité par 30 cm3 d'acide chlorhydrique à 37% dans un mélange d'acide acétique (30 cm3) et d'eau distillée (20 cm3). Le mélange est chauffé 3 heures à ébullition. Après refroidissement à une température voisine de 20°C et addition à 100 cm3 d'eau distillée, la solution aqueuse est neutralisée par de la soude à 30% et la phase organique extraite par de l'acétate d'éthyle. On obtient 4,0g de phényl-4 [(trifluorométhoxy-4 anilino)-2 éthyl]-1 pipéridine sous forme d'une huile brune utilisée à l'état brut dans la réaction suivante.

EXEMPLE 15

On opère comme à l'exemple 1 mais à partir de 0,6 g de {[(fluoro-2 phényl)-4 pipérazinyl-1]-2 éthyl}-3 trifluoroacétylimino-2 trifluorométhoxy-6 benzothiazoline, de 20 cm3 de méthanol et de 10 cm3 d'une solution

hydroalcoolique à 7% de carbonate de potassium. Après addition d'éther chlorhydrique 4,2N, on obtient 0,335g de dichlorhydrate d'imino-2 {[(fluoro-2 phényl)-4 pipérazinyl-1]-2 éthyl}-3 trifluorométhoxy-6 benzothiazoline fondant vers 260°C.

La {[(fluoro-2 phényl)-4 pipérazinyl-1]-2 éthyl}-3 trifluoroacétylimino-2 trifluorométhoxy-6 benzothiazoline peut être préparée de la façon suivante : on opère comme à l'exemple 1 pour la préparation de la {[(pyridyl-2)-4 pipérazinyl-1]-2 éthyl}-3 trifluoroacétylimino-2 trifluorométhoxy-6 benzothiazoline, à partir de 4,52g de méthane sulfonate de (trifluoroacétylimino-2 trifluorométhoxy-6 benzothiazolinyl-3)-2 éthyle dans 70cm3 de toluène et de 9g de (fluoro-2 phényl)-4 pipérazine. On obtient après purification par chromatographie sur colonne de silice avec de l'acétate d'éthyle comme éluant, 0,6g de {[(fluoro-2 phényl)-4 pipérazinyl-1]-2 éthyl}-3 trifluoroacétylimino-2 trifluorométhoxy-6 benzothiazoline.

EXEMPLE 16

On opère comme à l'exemple 1, à partir de 4,52g de méthane sulfonate de (trifluoroacétylimino-2 trifluorométhoxy-6 benzothiazolinyl-3)-2 éthyle dans 120 cm3 de toluène et de 7,8g de (chloro-3 phényl)-4 pipérazine. Après 2 heures au reflux, le filtrat toluènique est évaporé sous pression réduite et 100 cm3 de méthanol et 10 cm3 d'une solution hydroalcoolique de carbonate de potassium à 7% sont ajoutés au résidu toluènique. Le mélange est agité à environ 20°C. Le méthanol est évaporé à pression réduite et le résidu est purifié par chromatographies sur colone de silica avec un mélange de dichlorméthane et de méthanol (95-5 en volumes) comme éluant puis avec de l'acétate d'éthyle comme éluant. Après addition d'éther chlorhydrique 4,2N, on obtient 0,9g de trichlorhydrate d'imino-2 {[(chloro-3 phényl)-4 pipérazinyl-1]-2 éthyl}-3 trifluorométhoxy-6 benzothiazoline fondant vers 260°C.

EXEMPLE 17

On opère comme à l'exemple 1, à partir de 4,52g de méthane sulfonate de (trifluoroacétylimino-2 trifluorométhoxy-6 benzothiazolinyl-3)-2 éthyle dans 120 cm3 de toluène et de 6,7g de (fluoro-3 phényl)-4 pipérazine. Après 2 heures au reflux, le filtrat toluènique est évaporé sous pression réduite et 100 cm3 de méthanol et 20 cm3 d'une solution hydroalcoolique de carbonate de potassium à 7% sont ajoutés au résidu toluènique. Après 2 heures d'agitation à environ 20°C le méthanol est évaporé à pression réduite et le résidu est purifié par chromatographies sur colonne de silice avec un mélange de dichlorométhane et de méthanol (98-2 en volumes) comme éluant puis de l'acétate d'éthyle. Après addition d'éther chlorhydrique 4,2N, on obtient 1g de dichlorhydrate d'imino-2 {[(fluoro-3 phényl)-4 pipérazinyl-1]-2 éthyl}-3 trifluorométhoxy-6 benzothiazoline fondant vers 270°C.

EXEMPLE 18

On opère comme à l'exemple 1 mais à partir de 1,8g du chlorhydrate de {[(méthoxy-2 phényl)-4 pipérazinyl-1]-2 éthyl}-3 trifluoroacétylimino-2 trifluorométhoxy-6 benzothiazoline dans 39 cm3 de méthanol et de 23 cm3 d'une solution hydroalcoolique à 7% de carbonate de potassium. Après purification par chromatographie sur colonne de silice avec de l'acétone comme éluant puis addition d'éther chlorhydrique 4,2 N, on obtient 1,2 g du trichlorhydrate de l'imino-2 {[méthoxy-2 phényl)-4 pipérazinyl-1]-2 éthyl}-3 trifluorométhoxy-6 benzothiazoline fondant à 210°C.

Le chlorhydrate de {[méthoxy-2 phényl)-4 pipérazinyl-1]-2 éthyl}-3 trifluoroacétylimino-2 trifluorométhoxy-6 benzothiazoline, peut être préparé de la façon suivante : on opère comme à l'exemple 1, à partir de 4,52g de méthane sulfonate de (trifluoroacétylimino-2 trifluorométhoxy-6 benzothiazolinyl-3)-2 éthyle dans 120 cm3 de toluène et de 7,7g de (méthoxy-2 phényl)-4 pipérazine. Après addition d'éther chlorhydrique 4,2N, on obtient 1,8g de chlorhydrate de {[méthoxy-2 phényl)-4 pipérazinyl-1]-2 éthyl}-3 trifluoroacétylimino-2 trifluorométhoxy-6 benzothiazoline fondant à 230°C.

La présente invention concerne également les médicaments constitués par un composé de formule (I) ou un sel d'un tel composé à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale , parentérale, rectale ou topique.

Comme compositions solides pour administration orale, peuvent être utilisés dese comprimés, des pilules, dese poudres, (capsules de gélatine, cachets) ou des granulés. Dans ces compositions, le principe actif selon l'invention est mélangé à un ou plusieurs diluants inertes, tels que amidon, cellulose, saccharose, lactose ou silice.

Ces compositions peuvent également comprendre des substances autres que des diluants, par exemple

un ou plusieurs lubrifiants tels que le stéarate de magnésium ou le talc, un colorant, un enrobage (dragées) ou un vernis.

Comme compositions liquide pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants, ou stabilisants.

Les compositions stériles par administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humain, les composés selon l'invention sont particulièrement utiles dans le traitement et la prévention des phénomènes convulsifs, des troubles schizophréniques et notamment des formes déficitaires de la schizophrénie, des troubles du sommeil, des phénomènes liés à l'ischémie cérébrale ainsi que des affections neurologiques où le glutamate peut être impliqué telles que la maladie d'Alzheimer, la maladie d'Huntington, la sclérose amyotrophique latérale et l'atrophie olivopontocérébelleuse.

Les doses dépendent de l'effet recherché, de la durée du traitement et de la voie d'administration utilisée ; elles sont généralement comprises entre 30 et 300 mg par jour par voie orale pour un adulte avec des doses unitaires allant de 10 à 100 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

EXEMPLE A

On prépare, selon la technique habituelle, des gélules dosées à 50 mg de produit actif ayant la composition suivante :

| | |
|---|---|
| - imino-2 {[(pyridyl-2)-4 pipérazinyl-1]-2 éthyl}-3 trifluorométhoxy-6 benzothiazoline ........... | 50 mg |
| - Cellulose .................................... | 18 mg |
| - Lactose ...................................... | 55 mg |
| - Silice colloïdale ............................ | 1 mg |
| - Carboxyméthylamidon sodique .................. | 10 mg |
| - Talc ......................................... | 10 mg |
| - Stéarate de magnésium ........................ | 1 mg |

EXEMPLE B

On prépare, selon la technique habituelle, des comprimés dosés à 50 mg de produit actif ayant la composition habituelle :

| | |
|---|---|
| - imino-2 {[(méthyl-4 phényl)-4 pipérazinyl-1]-2 éthyl}-3 trifluorométhoxy-6 benzothiazoline .. | 50 mg |
| - Lactose .................................... | 104 mg |
| - Cellulose .................................... | 40 mg |
| - Polyvidone .................................... | 10 mg |
| - Carboxyméthylamidon sodique .................. | 22 mg |
| - Talc .......................................... | 10 mg |
| - Stéarate de magnésium ........................ | 2 mg |
| - Silice colloïdale .......................... | 2 mg |
| - Mélange d'hydroxyméthylcellulose, glycérine, oxyde de titane (71-3,5-24,5) .........q.s.p. | 1 comprimé pelliculé terminé à 245 mg |

EXEMPLE C

On prépare une solution injectable contenant 10 mg de produit actif ayant la composition suivante :

| | |
|---|---|
| - [(benzyl-4 pipérazinyl-1)-2 éthyl]-3 imino-2 trifluorométhoxy-6 benzothiazoline ......... | 10 mg |
| - Acide benzoïque ............................ | 80 mg |
| - Alcool benzylique .......................... | 0,06 $cm^3$ |
| - Benzoate de sodium ......................... | 80 mg |
| - Ethanol à 95 % ............................. | 0,4 $cm^3$ |
| - Hydroxyde de sodium ........................ | 24 mg |
| - Propylène glycol ........................... | 1,6 $cm^3$ |
| - Eau ...................................q.s.p. | 4 $cm^3$ |

**Revendications**

**Revendications pour les Etats contractants suivants : AT, BE, CH, DE, DK, FR, GB, IT, LI, LU, NL, SE**

**1.** Composés de formule :

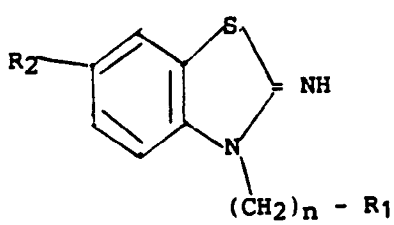

(I)

dans laquelle

- $R_1$ représente
  . un radical pipérazinyl-1 substitué en position -4 par (a) un radical phényle, (b) un radical phényle substitué par au moins un substituant choisi parmi les atomes d'halogène, les radicaux alkyle et alcoxy, (c) un radical phénylalkyle, (d) un radical pyridyle ou (e) un radical pyrimidinyle,
  . un radical tétrahydro-1,2,3,6 pyridyl-1 substitué en position -4 par un radical phényle ou phényle substitué par au moins un substituant choisi parmi les atomes d'halogène, les radicaux alkyle et alcoxy,
  . un radical pipéridino substitué en position -4 par un radical phényle ou phényle substitué par au moins un substituant choisi parmi les atomes d'halogène, les radicaux alkyle et alcoxy,
- $R_2$ représente un radical polyfluoroalcoxy,
- n est égal à 2 ou 3,

étant entendu que les radicaux alkyle et alcoxy et les portions alkyle et alcoxy contiennent 1 à 4 atomes de carbone en chaîne droite ou ramifiée,
ainsi que leurs sels avec un acide minéral ou organique

2. Composés de formule (I) selon la revendication 1 pour lesquels $R_2$ représente un radical trifluorométhoxy, pentafluoroéthoxy, trifluoro-2,2,2 éthoxy ou tétrafluoro-1,1,2,2 éthoxy.

3. Procédé de préparation des composés de formule (I) selon la revendication 1 caractérisé en ce que l'on hydrolyse un dérivé de formule :

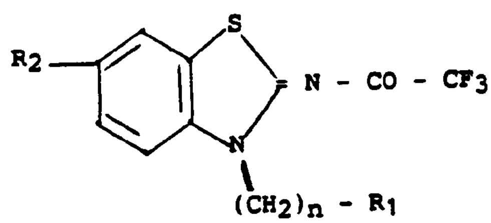

(II)

dans laquelle $R_1$, $R_2$ et n ont les mêmes significations que dans la revendication 1, isole le produit et le transforme éventuellement en sel d'addition avec un acide minéral ou organique.

4. Procédé de préparation des composés de formule (I) selon la revendication 1 caractérisé en ce que l'on fait réagir du brome et un thiocyanate de métal alcalin sur un dérivé de formule :

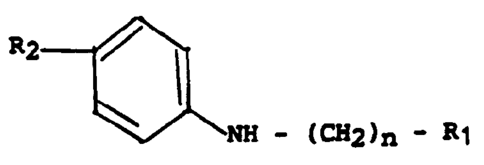

(VIII)

dans laquelle $R_1$, $R_2$ et n ont les mêmes significations que dans la formule (I), isole le produit et le transforme éventuellement en sel d'addition avec un acide minéral ou organique.

5. Médicaments caractérisés en ce qu'ils contiennent en tant que principe actif au moins un composé selon la revendication 1 ou un sel d'un tel composé.

6. Médicaments caractérisés en ce qu'ils contiennent en tant que principe actif au moins un composé selon la revendication 2 où un sel d'un tel composé.

7. Médicaments selon la revendication 5 et 6 pour le traitement des affections où le glutamate est impliqué.

**Revendications pour les Etats contractants suivants : ES, GR**

1. Procédé de préparation des composés de formule :

(I)

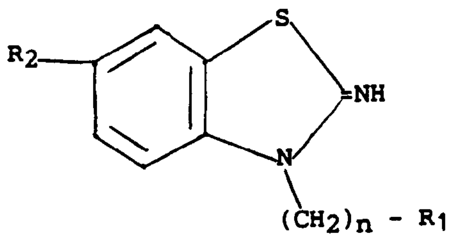

dans laquelle

- $R_1$ représente
  - . un radical pipérazinyl-1 substitué en position -4 par (a) un radical phényle, (b) un radical phényle substitué par au moins un substituant choisi parmi les atomes d'halogène, les radicaux alkyle et alkoxy, (c) un radical phénylalkyle, (d) un radical pyridyle ou (e) un radical pyrimidinyle,
  - . un radical tétrahydro-1, 2, 3, 6 pyridyl-1 substitué en position-4 par un radical phényle ou phényle substitué par au moins un substituant choisi parmi les atomes d'halogène, les radicaux alkyle et alcoxy,
  - . un radical pipéridino substitué en position -4 par un radical phényle ou phényle substitué par au moins un substituant choisi parmi les atomes d'halogène, les radicaux alkyle et alcoxy,
- $R_2$ représente un radical polyfluoroalcoxy,
- n est égal à 2 ou 3,

étant entendu que les radicaux alkyle et alcoxy et les portions alkyle et alcoxy contiennent 1 à 4 atomes de carbone en chaîne droite ou ramifiée,

ainsi que leurs sels avec un acide minéral ou organique, caractérisé en ce que :

A - ou bien on hydrolyse un dérivé de formule:

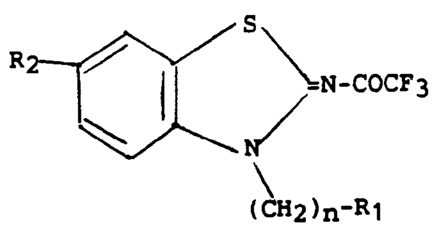

dans laquelle $R_1$, $R_2$ et n ont les mêmes significations que dans la formule (I), isole le produit et le transforme éventuellement en sel d'addition avec un acide minéral ou organique,

B - ou bien on fait réagir du brome et un thiocyanate de métal alcalin sur un dérivé de formule :

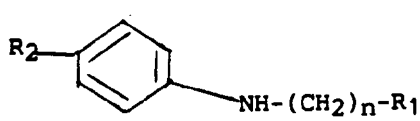

(VII)

dans laquelle $R_1$, $R_2$ et n ont les mêmes significations que dans la formule (I), isole le produit et le transforme éventuellement en sel d'addition avec un acide minéral ou organique.

**2.** Procédé selon la revendiquation 1 pour la préparation des composés de formule (I) pour lesquels $R_2$ représente un radical trifluorométhoxy, pentafluoroéthoxy, trifluoro-2, 2, 2 éthoxy ou tétrafluoro-1, 1, 2, 2 éthoxy.

### Patentansprüche

### Patentansprüche für folgende Vertragsstaaten : AT, BE, CH, DE, DK, FR, GB, IT, LI, LU, NL, SE

**1.** Verbindungen der Formel

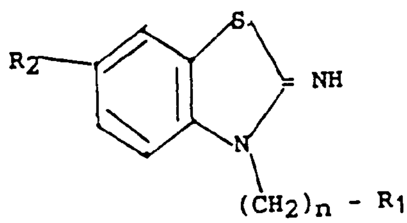

(I)

worin

- $R_1$ bedeutet:
  . einen Piperazin-1-ylrest, substituiert in 4-Stellung durch (a) einen Phenylrest, (b) einen Phenylrest, substituiert durch zumindest einen Substituenten, ausgewählt unter den Halogenatomen, den Alkyl- und Alkoxyresten, (c) einen Phenylalkylrest, (d) einen Pyridylrest oder (e) einen Pyrimidinylrest,
  . einen 1,2,3,6-Tetrahydro-pyrid-1-ylrest, substituiert in 4-Stellung durch einen Phenylrest, oder einen Phenylrest, substituiert durch zumindest einen Substituenten, ausgewählt unter den Halogenatomen, den Alkyl- und Alkoxyresten,
  . einen Piperidinorest, substituiert in 4-Stellung durch einen Phenylrest, oder einen Phenylrest, substituiert durch zumindest einen Substituenten, ausgewählt unter den Halogenatomen, den Alkyl- und Alkoxyresten,
- $R_2$ einen Polyfluoralkoxyrest bedeutet,
- n für 2 oder 3 steht,

mit der Maßgabe, daß die Alkyl- und Alkoxyreste und die Alkyl- und Alkoxyteile 1 bis 4 Kohlenstoffatome in gerader oder verzweigter Kette enthalten,
sowie deren Salze mit einer Mineral- oder organischen Säure.

**2.** Verbindungen der Formel (I) gemäß Anspruch 1, worin $R_2$ einen Trifluormethoxy-, Pentafluorethoxy-, 2,2,2-Trifluorethoxy- oder 1,1,2,2-Tetrafluorethoxyrest bedeutet.

**3.** Verfahren zur Herstellung der Verbindungen der Formel (I) gemäß Anspruch 1, dadurch gekennzeichnet, daß man ein Derivat der Formel

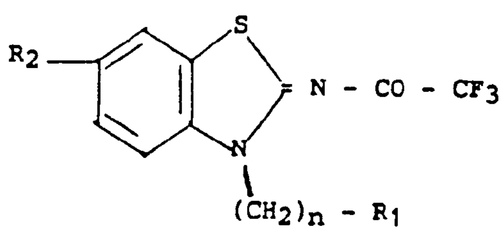

(II)

worin $R_1$, $R_2$ und n die in Anspruch 1 angegebenen Bedeutungen besitzen, hydrolisiert, das Produkt isoliert und es gegebenenfalls in ein Additionssalz mit einer Mineral- oder organischen Säure überführt.

**4.** Verfahren zur Herstellung der Verbindungen der Formel (I) gemäß Anspruch 1, dadurch gekennzeichnet, daß man Brom und ein Alkalimetallthiocyanat mit einem Derivat der Formel

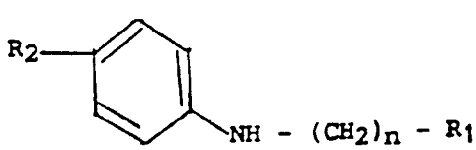

(VIII)

worin $R_1$, $R_2$ und n die für die Formel (I) angegebenen Bedeutungen besitzen, umsetzt, das Produkt isoliert und es gegebenenfalls in ein Additionssalz mit einer Mineral- oder organischen Säure überführt.

**5.** Arzneimittel, dadurch gekennzeichnet, daß sie als Wirkstoff zumindest eine Verbindung gemäß Anspruch

1 oder ein Salz einer derartigen Verbindung enthalten.

6. Arzneimittel, dadurch gekennzeichnet, daß sie als Wirkstoff zumindest eine Verbindung gemäß Anspruch 2 oder ein Salz einer derartigen Verbindung enthalten.

7. Arzneimittel gemäß Anspruch 5 und 6 für die Behandlung von gesundheitlichen Schädigungen, bei denen Glutamat eine Rolle spielt.

**Patentansprüche für folgenden Vertragsstaaten : ES, GR**

1. Verfahren zur Herstellung der Verbindungen der Formel

(I)

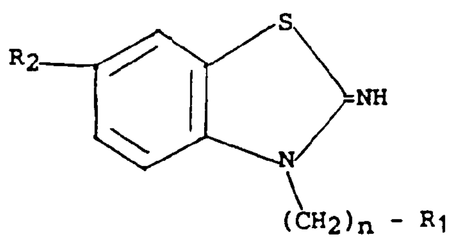

worin
- $R_1$ bedeutet:
  . einen Piperazin-1-ylrest, substituiert in 4-Stellung durch (a) einen Phenylrest, (b) einen Phenylrest, substituiert durch zumindest einen Substituenten, ausgewählt unter den Halogenatomen, den Alkyl- und Alkoxyresten, (c) einen Phenylalkylrest, (d) einen Pyridylrest oder (e) einen Pyrimidinylrest,
  . einen 1,2,3,6-Tetrahydro-pyrid-1-ylrest, substituiert in 4-Stellung durch einen Phenylrest, oder einen Phenylrest, substituiert durch zumindest einen Substituenten, ausgewählt unter den Halogenatomen, den Alkyl- und Alkoxyresten,
  . einen Piperidinorest, substituiert in 4-Stellung durch einen Phenylrest, oder einen Phenylrest, substituiert durch zumindest einen Substituenten, ausgewählt unter den Halogenatomen, den Alkyl- und Alkoxyresten,
- $R_2$ einen Polyfluoralkoxyrest bedeutet,
- n für 2 oder 3 steht,

mit der Maßgabe, daß die Alkyl- und Alkoxyreste und die Alkyl- und Alkoxyteile 1 bis 4 Kohlenstoffatome in gerader oder verzweigter Kette enthalten,
sowie von deren Salzen mit einer Mineral- oder organischen Säure, dadurch gekennzeichnet, daß man
A - entweder ein Derivat der Formel

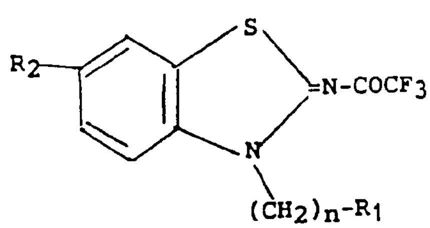

worin $R_1$, $R_2$ und n die für Formel (I) angegebenen Bedeutungen besitzen, hydrolysiert, das Produkt isoliert und es gegebenenfalls in ein Additionssalz mit einer Mineral- oder organischen Säure überführt,
B - oder Brom und ein Alkalimetallthiocyanat mit einem Derivat der Formel

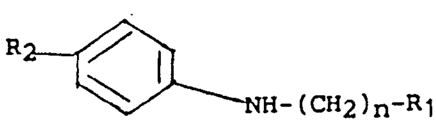

(VII)

worin $R_1$, $R_2$ und n die für Formel (I) angegebenen Bedeutungen besitzen, umsetzt, das Produkt isoliert und es gegebenenfalls in ein Additionssalz mit einer Mineral- oder organischen Säure überführt.

2. Verfahren gemäß Anspruch 1 zur Herstellung von Verbindungen der Formel (I), worin $R_2$ einen Trifluormethoxy-, Pentafluorethoxy-, 2,2,2-Trifluorethoxy-, oder 1,1,2,2-Tetrafluorethoxyrest bedeutet.

## Claims

### Claims for the following Contracting States : AT, BE, CH, DE, DK, FR, GB, IT, LI, LU, NL, SE

1. Compounds of formula:

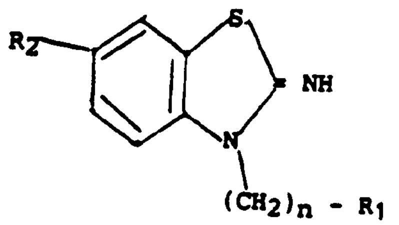

(I)

in which

- $R_1$ represents
  - . a 1-piperazinyl radical substituted in the 4-position by (a) a phenyl radical, (b) a phenyl radical substituted by at least one substituent chosen from halogen atoms or alkyl and alkoxy radicals, (c) a phenylalkyl radical, (d) a pyridyl radical or (e) a pyrimidinyl radical,
  - . a 1,2,3,6-tetrahydro-1-pyridyl radical substituted in the 4-position by a phenyl radical or a phenyl radical substituted by at least one substituent chosen from halogen atoms or alkyl and alkoxy radicals,
  - . a piperidino radical substituted in the 4-position by a phenyl radical or a phenyl radical substituted by at least one substituent chosen from halogen atoms or alkyl and alkoxy radicals,
- $R_2$ represents a polyfluoroalkoxy radical,
- n is equal to 2 or 3,

it being understood that the alkyl and alkoxy radicals and the alkyl and alkoxy portions contain 1 to 4 carbon atoms in a straight or branched chain,
and their salts with an inorganic or organic acid.

2. Compounds of formula (I) according to claim 1, for which $R_2$ represents a trifluoromethoxy, pentafluoroethoxy, 2,2,2-trifluoroethoxy or 1,1,2,2-tetrafluoroethoxy radical.

3. Process for the preparation of the compounds of formula (I) according to claim 1, characterized in that a derivative of formula:

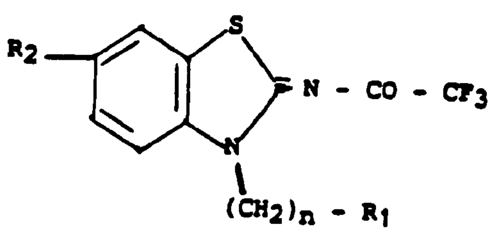

(II)

in which $R_1$, $R_2$ and n have the same meanings as in claim 1, is hydrolyzed, the product is isolated and optionally converted to an addition salt with an inorganic or organic acid.

4. Process for the preparation of the compounds of formula (I) according to claim 1, characterized in that bromine and an alkali metal thiocyanate are reacted with a derivative of formula:

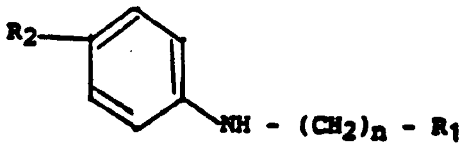

(VIII)

in which $R_1$, $R_2$ and n have the same meanings as in formula (I), the product is isolated and optionally converted to an addition salt with an inorganic or organic acid.

**5.** Medicaments, characterized in that they contain, as active principle, at least one compound according to claim 1 or a salt of such a compound.

**6.** Medicaments, characterized in that they contain, as active principle, at least one compound according to claim 2 or a salt of such a compound.

**7.** Medicaments according to claim 5 and 6 for the treatment of ailments where glutamate is involved.

**Claims for the following Contracting States : ES, GR**

**1.** Process for the preparation of the compounds of formula:

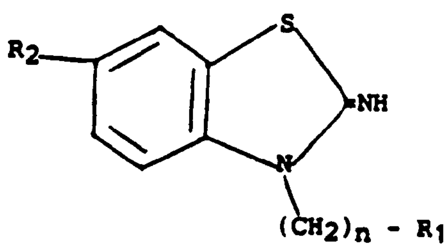

(I)

in which
. $R_1$ represents
. a 1-piperazinyl radical substituted in the 4-position by (a) a phenyl radical, (b) a phenyl radical substituted by at least one substituent chosen from halogen atoms or alkyl and alkoxy radicals, (c) a phenylalkyl radical, (d) a pyridyl radical or (e) a pyrimidinyl radical,
. a 1,2,3,6-tetrahydro-1-pyridyl radical substituted in the 4-position by a phenyl radical or a phenyl radical substituted by at least one substituent chosen from halogen atoms or alkyl and alkoxy radicals,
. a piperidino radical substituted in the 4-position by a phenyl radical or a phenyl radical substituted by at least one substituent chosen from halogen atoms or alkyl and alkoxy radicals,
. $R_2$ represents a polyfluoroalkoxy radical,
. n is equal to 2 or 3,
it being understood that the alkyl and alkoxy radicals and the alkyl and alkoxy portions contain 1 to 4 carbon atoms in a straight or branched chain,
and their salts with an inorganic or organic acid, characterized in that:
A - either a derivative of formula:

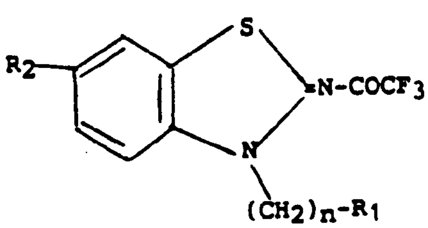

(II)

in which $R_1$, $R_2$ and n have the same meanings as in formula (I), is hydrolyzed, the product is isolated and optionally converted to an addition salt with an inorganic or organic acid,

B - or else bromine and an alkali metal thiocyanate are reacted with a derivative of formula:

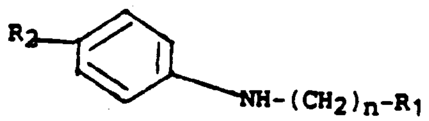

(VIII)

in which $R_1$, $R_2$ and n have the same meanings as in formula (I), the product is isolated and optionally converted to an addition salt with an inorganic or organic acid.

**2.** Process according to claim 1 for the preparation of the compounds of formula (I) for which $R_2$ represents a trifluoromethoxy, pentafluoroethoxy, 2,2,2-trifluoroethoxy or 1,1,2,2-tetrafluoroethoxy radical.